# FASCICULE DE BREVET EUROPEEN

(11) **EP 4 444 375 B1**
(45) Date de publication et mention de la délivrance du brevet: **08.04.2026**
(21) Numéro de dépôt: 22813678.4
(22) Date de dépôt: 03.11.2022
(51) Int. Cl.: A61M 1/00, C12M 1/00, C12M 1/36, C12M 1/33

(54) **MACHINE POUR LA PURIFICATION D'UN TISSU ADIPEUX ET L'INTRODUCTION DU TISSU ADIPEUX PURIFIE DANS LE CORPS D'UN PATIENT**
MASCHINE ZUR REINIGUNG VON FETTGEWEBE UND EINFÜHRUNG DES GEREINIGTEN FETTGEWEBES IN DEN KÖRPER EINES PATIENTEN
MACHINE FOR THE PURIFICATION OF ADIPOSE TISSUE AND INTRODUCTION OF THE PURIFIED ADIPOSE TISSUE INTO A PATIENT'S BODY

(30) Priorité: 08.12.2021 FR 2113137
(43) Date de publication de la demande: 16.10.2024
(73) Titulaire: Neosyad, 13290 Aix-en-Provence (FR)
(72) Inventeur: ROCHE, Régis, 83510 Lorgues (FR); CABAUD, François, 13510 Eguilles (FR); NELISSEN, Xavier, 4453 Villers Saint Siméon (BE)
(74) Mandataire: Cabinet Beau de Loménie
(86) Numéro de dépôt international: PCT/FR2022/052073
(87) Numéro de publication internationale: WO 2023/105129

(56) Documents cités:
- US-A1- 2008 281 256
- US-A1- 2017 304 509
- US-A1- 2020 061 259

## Description

### Domaine Technique

La présente invention concerne une machine pour la purification d'un tissu adipeux et l'introduction du tissu adipeux purifié dans le corps d'un patient, ainsi que des ensembles associés. L'invention trouve notamment une application pour les opérations de greffe autologue de tissu adipeux (désignées par « lipofilling » en langue anglaise) à visées esthétiques et reconstructrices. L'invention trouve, en particulier, une utilisation pour les opérations de chirurgie mammaire, la mise en œuvre de la machine objet de l'invention n'étant toutefois pas limitée à cette application.

### Technique antérieure

Des opérations de greffe autologue de tissu adipeux sont utilisées en chirurgie notamment en chirurgie mammaire afin de modeler le sein pour lui donner un aspect plus naturel après une perforante de l'artère épigastrique profonde intérieure (« Deep Inferior Epigastric Perforator », DIEP), une reconstruction par lambeau de grand dorsal, ou après le placement d'un implant mammaire. Ces opérations consistent à prélever de la graisse d'un patient dans une zone donneuse pour la réintroduire ensuite dans la zone d'intérêt du corps du patient. Ces opérations n'ont actuellement pas un rendu reproductible et peuvent nécessiter plusieurs interventions du fait de la résorption de la matière introduite (perte de volume rencontrée après l'opération). En outre, la préparation de la graisse en vue de sa réintroduction est réalisée par un opérateur, chirurgien ou assistant du chirurgien, ce qui engendre un coût de mise en œuvre de l'opération relativement élevé et un risque d'erreur ou de perte en reproductibilité. Il est donc souhaitable de proposer une technique qui simplifie et rende plus fiables les opérations existantes.

Le document US 2020/0054824 a cherché à proposer une amélioration par mise en œuvre d'un système en boucle fermée dans lequel la graisse est récupérée pour être préparée en vue de sa réintroduction en étant mélangée à un liquide de lavage dans un dispositif de filtration par gravité commercialisé sous la référence commerciale Revolve^{®}, et être ensuite réintroduite dans le corps du patient à l'aide d'une pompe centrifuge. Il est toutefois possible d'améliorer la qualité de la graisse réintroduite.

### Exposé de l'invention

La présente invention vise à surmonter les inconvénients de l'art antérieur et concerne une machine pour la purification d'un tissu adipeux et l'introduction du tissu adipeux purifié dans le corps d'un patient, la machine comprenant au moins :
- un circuit de circulation de matière comprenant au moins (i) un dispositif de purification apte à purifier un tissu adipeux prélevé du corps d'un patient, le dispositif de purification comprenant des moyens de rétention du tissu adipeux et de filtration des matières polluantes délimitant un volume de traitement destiné à accueillir le tissu adipeux, et un moyen d'entrainement en rotation apte à mettre les moyens de rétention et de filtration en rotation, et (ii) un dispositif d'introduction apte à être mis en communication avec le dispositif de purification et destiné à l'introduction du tissu adipeux purifié dans le corps du patient, le circuit étant en outre muni de moyens de contrôle de la circulation de matière aptes à autoriser ou empêcher la communication entre des éléments du circuit, et
- une unité de commande configurée pour actionner le dispositif de purification et les moyens de contrôle,
l'unité de commande étant configurée pour mettre le circuit dans une configuration de purification du tissu adipeux dans laquelle les moyens de rétention et de filtration sont mis en rotation pour purifier le tissu adipeux par centrifugation et filtration des matières polluantes, et l'unité de commande étant configurée pour mettre le circuit dans une configuration d'introduction du tissu adipeux purifié dans le corps du patient dans laquelle le tissu adipeux purifié est introduit depuis le dispositif de purification dans le dispositif d'introduction.

De manière caractéristique, l'invention met en œuvre une purification du tissu adipeux par centrifugation et filtration des matières polluantes au travers des moyens de rétention et de filtration du fait de la mise en rotation de ces derniers. Cela permet avantageusement d'optimiser la qualité du tissu adipeux purifié en réduisant la présence de liquide interstitiel et en s'affranchissant de la présence d'un organe de mélange ou de malaxage qui risquerait d'affecter l'intégrité des cellules adipeuses comme dans le cas du dispositif commercialisé sous la référence commerciale Revolve^{®} mis en œuvre dans US 2020/0054824. Le tissu adipeux ainsi purifié présente en particulier un taux de résorption bien inférieur à celui obtenu avec les solutions de purification de l'art antérieur. En particulier, les matières polluantes peuvent être sous la forme d'un milieu liquide comprenant l'un au moins de l'huile, du sang, de l'eau éventuellement dans un liquide de lavage. L'invention utilise une filtration assistée par centrifugation dans laquelle la séparation entre le tissu adipeux et les matières polluantes est facilitée par la mise en rotation des moyens de rétention et de filtration. Le tissu adipeux est soumis à des accélérations centrifuges contre la paroi interne des moyens de rétention et de filtration, permettant de drainer les matières polluantes à travers les pores de ces moyens alors que le tissu adipeux est retenu dans le volume de traitement. La solution selon l'invention se distingue également des techniques de centrifugation en centrifugeuse où le tissu adipeux et les matières polluantes sont présentes dans un contenant totalement fermé et qui mettent en œuvre des accélérations très importantes, généralement supérieures à 400 G, pour obtenir une séparation de phases et peuvent conduire à un endommagement du tissu adipeux. En outre, par rapport à ces techniques, la purification selon l'invention permet d'éliminer l'huile présente dont la réintroduction dans le corps du patient doit être évitée car elle peut former des kystes huileux.

Selon l'invention telle que revendiquée, le circuit comprend, en outre, un segment d'entrée destiné au prélèvement du tissu adipeux du corps du patient qui est relié au dispositif de purification et une pompe de prélèvement configurée pour introduire le tissu adipeux prélevé depuis le segment d'entrée dans le dispositif de purification, le circuit étant apte à définir une boucle de circulation fermée entre le segment d'entrée et le dispositif d'introduction avec le corps du patient,
l'unité de commande étant configurée pour actionner la pompe de prélèvement et mettre le circuit dans une configuration de prélèvement du tissu adipeux du corps du patient dans laquelle le tissu adipeux prélevé est introduit dans le dispositif de purification par la pompe de prélèvement, et retenu par les moyens de rétention et de filtration dans le dispositif de purification.

Une telle caractéristique permet avantageusement que la machine forme un circuit fermé avec le patient qui empêche une mise à l'air libre du tissu adipeux prélevé, évitant ainsi tout risque de contamination.

Selon l'invention telle que revendiquée, le segment d'entrée est apte à communiquer avec une entrée d'aspiration du dispositif de purification et le circuit comprend, en outre, un réservoir de récupération relié à au moins une sortie d'aspiration du dispositif de purification et apte à être mis en communication avec la pompe de prélèvement, l'unité de commande étant configurée pour mettre le circuit dans une configuration de purification dans laquelle des matières polluantes du tissu adipeux sont aspirées par la pompe de prélèvement au travers de ladite au moins une sortie d'aspiration du dispositif de purification et récupérées dans le réservoir de récupération.

Une telle caractéristique participe à augmenter davantage encore la compacité de la machine en utilisant la même pompe pour les opérations de prélèvement du tissu adipeux et de récupération des matières polluantes.

En particulier, les moyens de rétention et de filtration peuvent être présents entre le volume de traitement et un volume d'évacuation distinct du volume de traitement, et le réservoir de récupération peut être relié à une première et une deuxième sorties d'aspiration du dispositif de purification, la première sortie d'aspiration débouchant dans le volume de traitement et la deuxième sortie d'aspiration débouchant dans le volume d'évacuation.

Une telle configuration mettant en œuvre plusieurs sorties d'aspiration dans le dispositif permet de faciliter les étapes de prélèvement du tissu adipeux et d'évacuation des matières polluantes.

Selon l'invention telle que revendiquée, le réservoir de récupération est relié au segment d'entrée par un segment de dérivation, placé en dérivation par rapport au dispositif de purification, et apte à être mis en communication avec la pompe de prélèvement, l'unité de commande étant configurée pour mettre le circuit dans une configuration de prélèvement d'un excédent de tissu adipeux du corps du patient dans laquelle la pompe de prélèvement aspire l'excédent de tissu adipeux au travers du segment de dérivation pour le récupérer dans le réservoir de récupération.

Une telle caractéristique permet avantageusement que la machine réalise une lipoaspiration supplémentaire du patient avec prélèvement d'un excédent de tissu adipeux par rapport à la quantité nécessaire pour la réintroduction dans le corps. Dans ce cas, cet excédent n'est pas destiné à être récupéré dans le dispositif de purification mais à être dirigé dans le réservoir de récupération après traversée du segment de dérivation.

Dans un exemple de réalisation, le circuit comprend en outre un contenant de stockage destiné à contenir un liquide de lavage du tissu adipeux, et une pompe de circulation apte à mettre en communication le contenant de stockage avec le dispositif de purification,
l'unité de commande étant configurée pour mettre le circuit dans une configuration de lavage du tissu adipeux dans laquelle le liquide de lavage est introduit depuis le contenant de stockage dans le dispositif de purification par la pompe de circulation. Une telle caractéristique permet d'intégrer directement au circuit de la machine le liquide de lavage, améliorant ainsi la compacité et garantissant que l'étape de lavage n'apporte pas de contaminants extérieurs.

Dans un exemple de réalisation, le circuit comprend en outre un contenant de stockage additionnel destiné à contenir un anesthésiant, la pompe de circulation étant apte à mettre en communication le contenant de stockage additionnel avec un segment de sortie destiné à l'introduction de matière dans le corps du patient, l'unité de commande étant configurée pour mettre le circuit dans une configuration d'anesthésie dans laquelle l'anesthésiant est introduit depuis le contenant de stockage additionnel dans le segment de sortie par la pompe de circulation.

Une telle caractéristique permet de fonctionnaliser davantage encore la machine, celle-ci permettant de réaliser l'anesthésie préalable au prélèvement, tout en conservant une solution compacte en utilisant la pompe de circulation, également utilisée pour le lavage du tissu adipeux.

Dans un exemple de réalisation, la pompe de circulation est une pompe à écoulement réversible apte à mettre en communication le contenant de stockage avec le dispositif de purification selon un premier sens d'écoulement, et le contenant de stockage additionnel avec le segment de sortie selon un deuxième sens d'écoulement différent du premier sens d'écoulement.

Dans un exemple de réalisation, la pompe de circulation est une pompe péristaltique.

Une telle caractéristique permet avantageusement de maintenir avec une grande précision le débit de matière circulante. En outre, seul l'intérieur du tube de la pompe péristaltique est en contact avec le produit pompé, ce qui permet d'éviter tout risque de contamination ou de détérioration de la pompe.

L'invention vise également un ensemble pour la purification d'un tissu adipeux et l'introduction du tissu adipeux purifié dans le corps d'un patient, comprenant au moins :
- une machine telle que décrite plus haut, et
- un dispositif chirurgical apte à introduire le tissu adipeux purifié dans le corps du patient relié au dispositif d'introduction.

Dans un exemple de réalisation, le circuit comprend en outre le segment d'entrée comme décrit plus haut, et l'ensemble comprend en outre un dispositif chirurgical apte à prélever le tissu adipeux relié au segment d'entrée.

### Brève description des dessins

[Fig. 1] La figure 1 est un ordinogramme montrant une succession d'étapes pouvant être mises en œuvre par une machine selon l'invention.
[Fig. 2] La figure 2 représente un exemple de machine selon l'invention pour laquelle le circuit est en configuration d'anesthésie.
[Fig. 3] La figure 3 représente l'exemple de machine selon l'invention pour laquelle le circuit est en configuration de prélèvement du tissu adipeux.
[Fig. 4] La figure 4 représente l'exemple de machine selon l'invention pour laquelle le circuit est en configuration de prélèvement d'un excédent de tissu adipeux.
[Fig. 5] La figure 5 représente l'exemple de machine selon l'invention pour laquelle le circuit est en configuration de lavage du tissu adipeux.
[Fig. 6] La figure 6 représente l'exemple de machine selon l'invention pour laquelle le circuit est en configuration d'aspiration des matières polluantes.
[Fig. 7] La figure 7 représente l'exemple de machine selon l'invention pour laquelle le circuit est en configuration d'introduction du tissu adipeux purifié dans le corps du patient.
[Fig. 8] La figure 8 représente une vue en perspective partielle d'un exemple de machine selon l'invention.
[Fig. 9] La figure 9 représente, de manière schématique et partielle, une variante de pompe de circulation pouvant être mise en œuvre dans le cadre de l'invention.
[Fig. 10] La figure 10 représente une variante de machine selon l'invention pour laquelle le chargement du dispositif d'introduction est effectué sans utiliser la pompe de circulation.
[Fig. 11] La figure 11 est une vue schématique éclatée d'un exemple de dispositif de purification utilisable dans le cadre de l'invention.
[Fig. 12] La figure 12 est une vue schématique en coupe du dispositif de purification de la figure 11 une fois assemblé.
[Fig. 13] La figure 13 est une vue schématique en coupe montrant le dispositif de purification de la figure 12 après déplacement du fond.

### Description des modes de réalisation

La suite s'attache à décrire, en lien avec les figures annexées, un exemple de machine 1 selon l'invention apte à mettre en œuvre un traitement en plusieurs étapes visant à introduire un tissu adipeux purifié dans le corps d'un patient pour une opération de greffe autologue de tissu adipeux, par exemple pour la chirurgie mammaire ou au niveau d'autres parties du corps. La description qui suit décrit ces étapes de manière chronologique ainsi que les configurations de la machine 1 associées à chacune d'entre elles. Sur chaque figure, seuls les symboles de référence des éléments pertinents pour le descriptif de l'action associée à la figure considérée sont indiqués afin de ne pas affecter la lisibilité. D'une manière générale et comme il sera davantage détaillé plus bas, la machine 1 comprend un circuit 2 de circulation de matière qui est contrôlé par une unité de commande 4 permettant la mise en œuvre des différentes étapes de traitement. Avant initiation du traitement le circuit 2 est dans un état stérile.

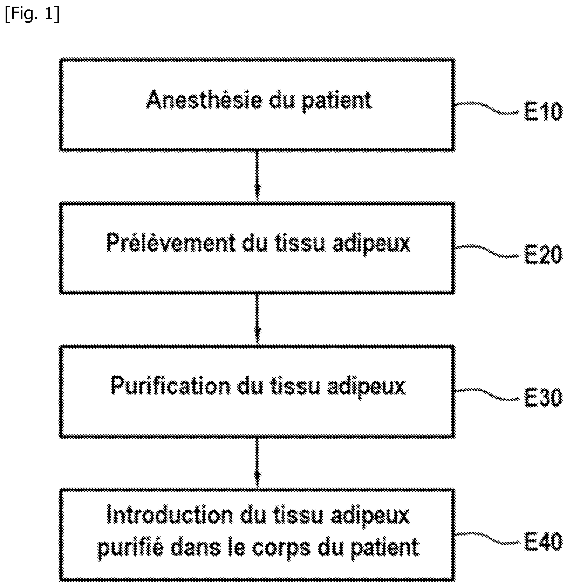

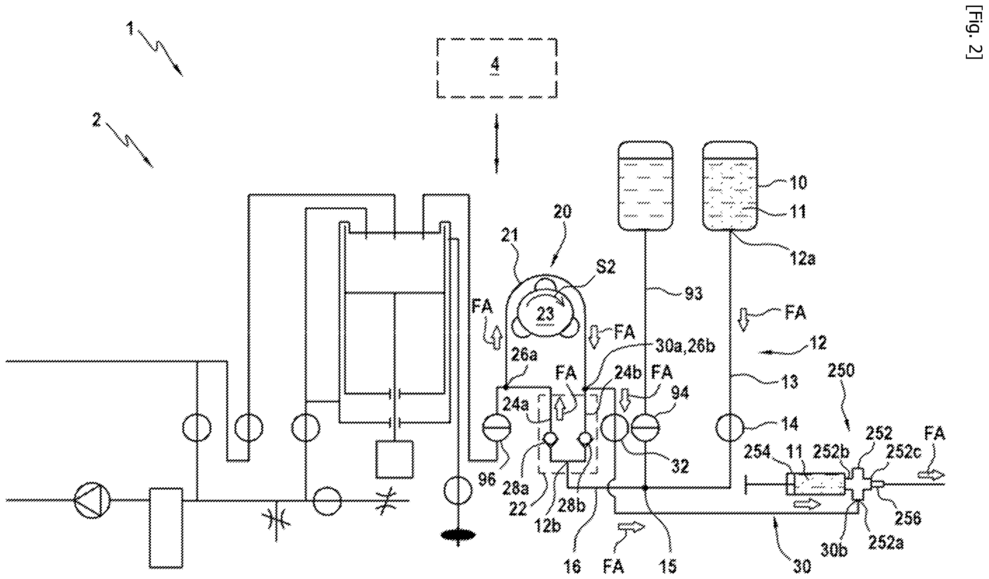

L'anesthésie (étape E10 sur la figure 1) est réalisée de manière préliminaire au prélèvement du tissu adipeux. L'exemple de machine 1 décrit en lien avec les figures 2 à 7 est apte à réaliser une anesthésie du patient et la configuration du circuit 2 correspondant à l'étape d'anesthésie est illustrée à la figure 2. Le circuit 2 comprend un contenant de stockage 10 contenant l'anesthésiant 11 lequel est sous forme liquide, par exemple sous la forme d'une solution aqueuse, une pompe 20 de circulation et un segment de sortie 30. L'anesthésiant peut être un anesthésiant local permettant la réalisation d'une anesthésie locale. Le contenant 10 peut être sous la forme d'une poche souple, par exemple en matière plastique. Le segment 30 est destiné à l'introduction de matière dans le corps du patient en étant relié à un dispositif d'introduction 250 apte à permettre cette introduction. Le dispositif 250 comprend un corps 252 muni d'un premier port 252a relié au segment 30, un deuxième port 252b relié à une seringue 254 et un troisième port 252c relié à une canule d'injection 256. Le premier port 252a est muni d'un clapet anti-retour de façon à permettre l'injection de l'anesthésiant 11 dans le patient au travers de la canule 256 par pression du piston de la seringue 254. Le chirurgien peut agir directement sur la seringue 254 afin de provoquer l'introduction de matière dans le corps du patient, ou en variante cette action peut être automatisée. Le dispositif d'introduction 250 est connu en soi, on peut par exemple utiliser un dispositif commercialisé sous la référence LipoGrafter^{®} par la société MTFBiologics.

La pompe 20 est ici une pompe péristaltique mais des variantes sont possibles comme décrit dans la suite. Le circuit 2 comprend un segment 12 reliant le contenant 10 à une entrée 22 de la pompe 20. Le segment 12 présente une première extrémité 12a reliée au contenant 10 et une deuxième extrémité 12b, distincte de la première extrémité 12a, reliée à l'entrée 22. Le segment 12 comprend ici deux conduits 13 et 16 dans le prolongement l'un de l'autre. Le conduit 16 prolonge le conduit 13 jusqu'à l'entrée 22 depuis un nœud 15 de liaison au niveau duquel les conduits 13, 16 et 93 (le conduit 93 sera décrit dans la suite) sont concourants. L'emploi d'un conduit 16 commun pour prolonger les conduits 13 et 93 est avantageux pour réduire la longueur de tubulure pour la liaison à la pompe 20, simplifiant ainsi le circuit 2. Selon une variante non illustrée, le conduit 93 n'est pas relié au conduit 13 et est connecté, de manière indépendante, à la pompe 20.

L'entrée 22 définit deux conduits 24a, 24b distincts d'alimentation s'étendant chacun jusqu'à un orifice distinct 26a, 26b du corps 21 de pompe 20. Chaque conduit 24a, 24b est muni d'un clapet anti-retour 28a, 28b. Le corps 21 de pompe 20 renferme le rotor 23 et les patins de la pompe 20 ainsi que le tuyau s'étendant entre les orifices 26a et 26b au travers duquel la matière est destinée à s'écouler. Le conduit 24a, respectivement 24b, s'étend entre le segment 12 (ou le conduit 16) et l'orifice 26a, respectivement 26b. Une première extrémité 30a du segment 30 s'étend depuis l'orifice 26b et une deuxième extrémité 30b du segment 30 est reliée au dispositif d'introduction 250.

Le segment 12 comprend un moyen de contrôle 14 de la circulation de matière qui est apte à autoriser ou empêcher le transfert de matière depuis le contenant 10 au travers de ce segment 12. En l'occurrence, le moyen 14 est présent sur le conduit 13 et permet d'autoriser ou d'empêcher la communication entre le contenant 10 et la pompe 20. Le segment 30 est également muni d'un moyen de contrôle 32 de la circulation de matière qui permet d'autoriser ou d'empêcher le transfert de matière depuis la pompe 20 au travers du segment 30. D'une manière générale, les segments et conduits mis en œuvre peuvent être en matériau souple, par exemple polymérique, et les moyens de contrôle de la circulation de matière peuvent être aptes à pincer les segments ou conduits (« pinch valve ») afin d'empêcher la circulation de matière et à relâcher le pincement afin d'autoriser cette circulation. Une telle solution est avantageuse afin de simplifier la conception du circuit mais l'homme du métier reconnaîtra que d'autres solutions sont possibles pour contrôler l'écoulement dans le circuit.

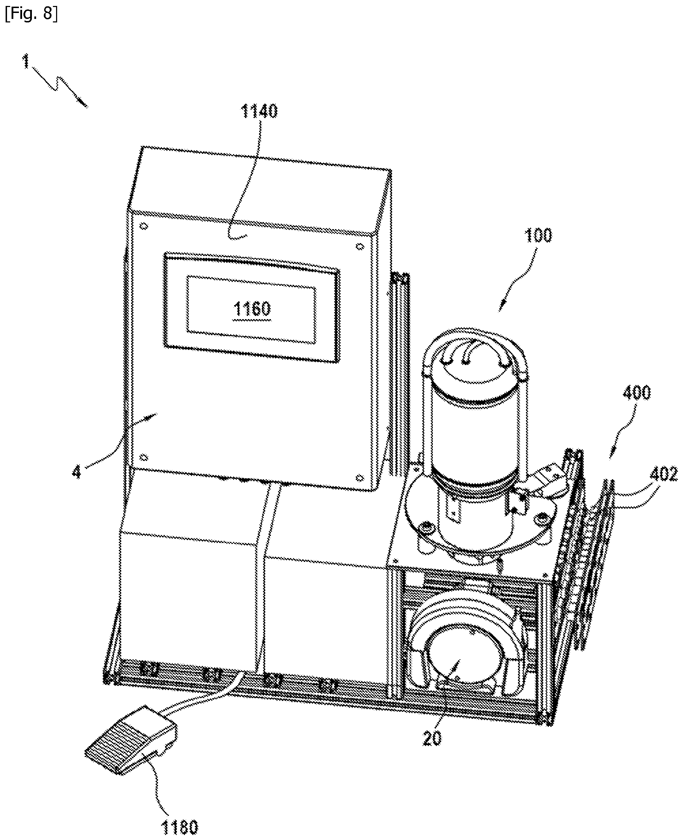

L'unité de commande 4 agit sur les moyens de contrôle de la circulation de matière pour autoriser ou empêcher la communication entre certains éléments du circuit en fonction de l'étape de traitement mise en œuvre par la machine 1. L'unité de commande 4 comprend généralement une interface de commande 1140 (voir figure 8) sur laquelle le chirurgien peut notamment sélectionner l'étape à réaliser en fonction de l'avancement de l'opération. L'interface de commande 1140 peut comporter un écran 1160, tactile ou non, permettant l'affichage de diverses informations, comme des informations relatives à la nature de l'étape de traitement en cours, à l'avancement de cette étape, des informations relatives au patient etc.

L'interface de commande 1140 comprend également au moins un élément d'interaction pour la commande de la machine 1 par le chirurgien, comprenant par exemple l'un au moins d'un ensemble de touches ou d'une pédale 1180. Le chirurgien choisit sur l'interface de commande l'étape à mettre en œuvre par la machine, cette information est ensuite transmise à un dispositif de contrôle de l'unité de commande 4 qui agit sur le circuit 2, en particulier sur la pompe 20 et les moyens de contrôle de la circulation de matière, pour mettre le circuit dans la configuration associée à l'étape de traitement sélectionnée. Le chirurgien peut alors initier l'étape de traitement souhaitée en agissant sur l'interface de commande, par exemple en pressant la pédale 1180. L'étape de traitement peut être réalisée sans interruption lorsque le chirurgien maintient une pression sur la pédale mais il peut également être souhaitable que l'étape soit réalisée de manière discontinue, avec une plusieurs interruptions, ces interruptions pouvant être provoquées par relâchement provisoire de la pédale. Selon une variante, l'étape de traitement peut être initiée par un appui du chirurgien sur la pédale puis la pression sur la pédale peut être relâchée durant la réalisation de l'étape et ensuite cette dernière peut être arrêtée par un nouvel appui du chirurgien sur la pédale. La machine 1 comprend également un élément de positionnement 400 des tubulures comprenant une pluralité de pistons 402 apte à pincer les segments ou conduits (« pinch valve ») afin d'empêcher la circulation de matière et à relâcher le pincement afin d'autoriser cette circulation. L'homme du métier reconnaîtra que d'autres solutions de pincement des tubulures sont envisageables.

Dans le cas de l'étape d'anesthésie E10, le dispositif de contrôle agit sur le circuit 2 de sorte à le mettre dans la configuration illustrée à la figure 2 puis l'anesthésie peut être lancée par pression de la pédale par le chirurgien. La pompe 20 est une pompe à écoulement réversible dans l'exemple illustré. Selon cette configuration, le dispositif de contrôle met les moyens 14 et 32 en configuration autorisant la circulation de matière (dite « configuration ouverte ») et actionne la pompe 20 pour qu'elle provoque un écoulement de matière le long du sens d'écoulement S2. Les moyens 94 et 96, qui seront décrits dans la suite, sont dans une configuration ne permettant pas la circulation de matière (dite « configuration fermée ») pendant l'anesthésie. De même, la pompe 60 et le dispositif de purification 100, évoqués plus bas, ne sont pas actionnés durant cette étape. Lors de l'anesthésie, le liquide 11 est aspiré par la pompe 20 au travers du segment 12 pour être refoulé dans le segment 30 et être ainsi introduit dans le corps du patient. Le liquide 11 s'écoule le long du sens d'écoulement S2 dans lequel il est introduit depuis le contenant 10 dans le segment 30 puis dans le dispositif 250 par la pompe 20. Le liquide 11 est ici aspiré par la pompe 20 au travers du conduit 24a et de l'orifice 26a pour être ensuite refoulé au travers de l'orifice 26b vers le segment 30. Les flèches FA matérialisent l'écoulement du liquide 11 dans le circuit 2 durant l'anesthésie ainsi que son injection dans le patient au travers de la canule 256.

Selon l'exemple illustré, la machine 1 est apte à réaliser l'anesthésie mais on notera que l'on ne sort pas du cadre de l'invention si ce n'est pas le cas, l'anesthésie étant réalisée sans utiliser la machine. Dans ce dernier cas, la machine peut, conformément à une variante non illustrée, être dépourvue de contenant 10, du segment 12 et du conduit 24a. On va maintenant décrire la suite du traitement consistant à prélever le tissu adipeux depuis le corps du patient (étape E20). Le tissu adipeux ainsi prélevé est destiné à être purifié en vue d'une réintroduction ultérieure dans le corps du patient.

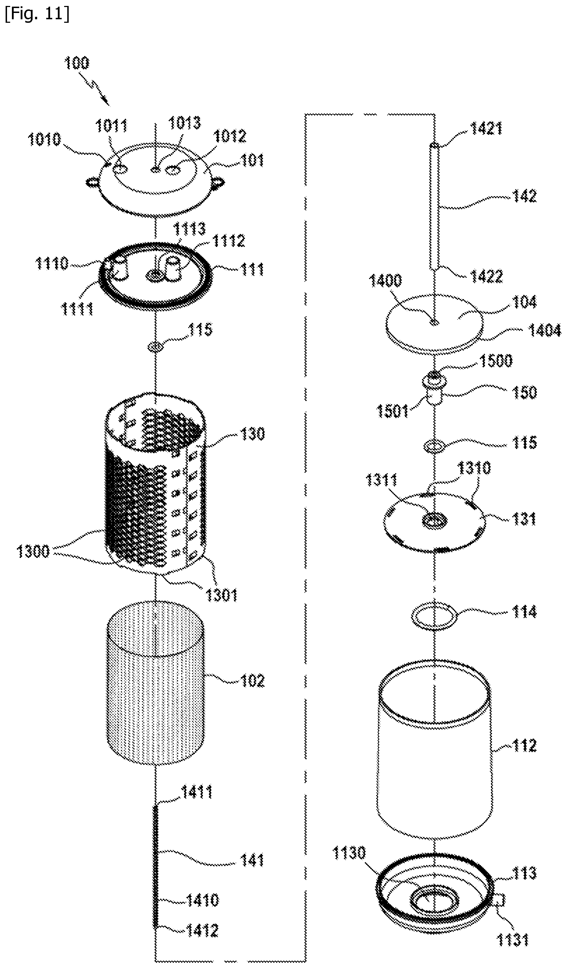

Le circuit 2 comprend un segment d'entrée 40 destiné au prélèvement du tissu adipeux du corps du patient en étant relié à un dispositif chirurgical (non représenté) apte à permettre ce prélèvement, tel qu'une canule d'aspiration. Le segment 40 relie le dispositif chirurgical de prélèvement à un dispositif de purification 100 qui est apte à purifier le tissu adipeux prélevé. Un exemple de dispositif 100 comprenant notamment des moyens 102 de rétention du tissu adipeux et de filtration des matières polluantes et sera décrit plus en détails dans la suite, en lien avec les figures 11 à 13. Les moyens 102 peuvent être sous la forme d'une paroi latérale filtrante. Les moyens 102 présentent des pores ayant une taille de pore configurée pour laisser passer les matières polluantes, par exemple sous forme d'un milieu liquide, et retenir le tissu adipeux. Le segment 40 comprend ici deux conduits 42 et 44 dans le prolongement l'un de l'autre. Le conduit 42 relie le dispositif chirurgical de prélèvement au nœud 45 de liaison au niveau duquel les conduits 42, 44 et 80 (le conduit 80 sera décrit dans la suite) sont concourants. Le conduit 44 s'étend entre une première extrémité 44a au niveau du nœud 45 et une deuxième extrémité 44b qui débouche dans le dispositif 100. La deuxième extrémité 44b, et donc le segment 40, débouche dans un volume V de traitement du dispositif 100 délimité par les moyens 102 et par un fond 104. Le segment 40 comprend un moyen de contrôle 46 de la circulation de matière qui est apte à autoriser ou empêcher le transfert de matière depuis le dispositif chirurgical de prélèvement au travers du segment 40 vers le dispositif 100. En l'occurrence, le moyen 46 est présent sur le conduit 44. Le circuit 2 comprend également un segment 50 qui relie le dispositif 100 à une pompe de prélèvement 60, distincte de la pompe 20 évoquée précédemment. La pompe 60 est une pompe d'aspiration. La pompe 60 peut être mise en communication avec le dispositif chirurgical de prélèvement au travers du segment 50, du dispositif 100 et du segment 40 pour aspirer le tissu adipeux du corps du patient. La pompe 60 peut être une pompe à vide. Le segment 50 comprend ici plusieurs extrémités 50a et 50b qui débouchent à l'intérieur du dispositif 100 pour effectuer une prise d'aspiration par la pompe de prélèvement 60 afin de créer une dépression dans le dispositif 100, ce qui permet d'aspirer le tissu adipeux depuis le corps du patient dans le dispositif 100 et ensuite d'évacuer les matières polluantes du tissu adipeux présent dans le dispositif 100. Selon l'exemple illustré, une première extrémité 50a du segment 50 débouche dans le volume V de traitement et une deuxième extrémité 50b du segment 50 débouche dans un volume VE d'évacuation du dispositif 100, distinct du volume V de traitement. L'extrémité 44b définit une entrée d'aspiration du dispositif 100 au travers de laquelle le tissu adipeux prélevé est destiné à être introduit dans le dispositif 100. Les extrémités 50a et 50b définissent une sortie d'aspiration du dispositif 100, en communication avec la pompe 60, au travers desquelles les matières polluantes du tissu adipeux sont destinées à être aspirées par la pompe 60. Les moyens 102 peuvent être situés entre les volumes V et VE. Le volume VE peut être situé autour des moyens 102 et du volume V, comme illustré. On a représenté un exemple à deux extrémités 50a et 50b mais on ne sort pas du cadre de l'invention si davantage d'extrémités sont présentes ou si seulement une extrémité est présente, tant que la dépression générée est suffisante pour permettre l'aspiration du tissu adipeux et l'évacuation des matières polluantes.

Le segment 50 comprend ici un nœud 55 de liaison au niveau duquel les conduits 52, 54 et 58 sont concourants. Le conduit 58 prolonge les conduits 52 et 54 au-delà du nœud 55 et comprend un moyen de contrôle 56 de la circulation de matière apte à mettre en communication la pompe 60 avec le dispositif 100 ou à empêcher cette communication. Le segment 50, en particulier le conduit 58, est relié à des régulateurs de vide 62 et 64 qui permettent de maintenir un niveau de vide prédéfini lors de l'aspiration par la pompe 60. Selon l'exemple illustré, une électrovanne 66 est présente entre le régulateur de vide 64 et le segment 50.

Le circuit 2 comprend également un conduit 70 ayant une première extrémité 70a débouchant dans le dispositif 100 (dans le volume VE dans l'exemple illustré) et une deuxième extrémité 70b débouchant à l'air libre et reliée à un filtre à air 74. La communication au travers du conduit 70 est contrôlée par le moyen de contrôle 72 de la circulation de matière.

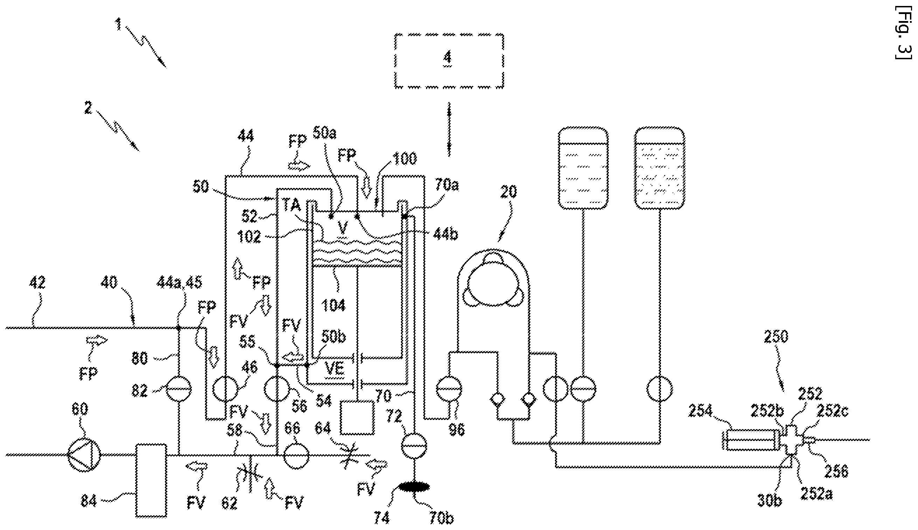

L'étape E20 de prélèvement du tissu adipeux peut être initiée par action du chirurgien sur l'interface de commande comme décrit plus haut. Le dispositif de contrôle agit sur le circuit 2 de sorte à le mettre dans la configuration illustrée à la figure 3 dans laquelle les moyens 46 et 56 sont en configuration ouverte, ainsi que l'électrovanne 66, et la pompe 60 actionnée. Les moyens 82 et 96, qui seront décrits dans la suite, et le moyen 72 sont en configuration fermée pendant cette étape. De même, la pompe 20 n'est pas actionnée. Durant le prélèvement, le tissu adipeux est aspiré depuis le corps du patient au travers du segment 40, par action de la pompe 60, pour être introduit dans le dispositif 100 au travers de l'extrémité 44b. Le tissu adipeux TA prélevé est alors retenu dans le dispositif 100 par les moyens 102. Les flèches FP matérialisent le transfert du tissu adipeux TA prélevé dans le circuit 2. Les flèches FV matérialisent le tirage du vide produit par la pompe 60 en aval du dispositif 100 pour aspirer le tissu adipeux TA.

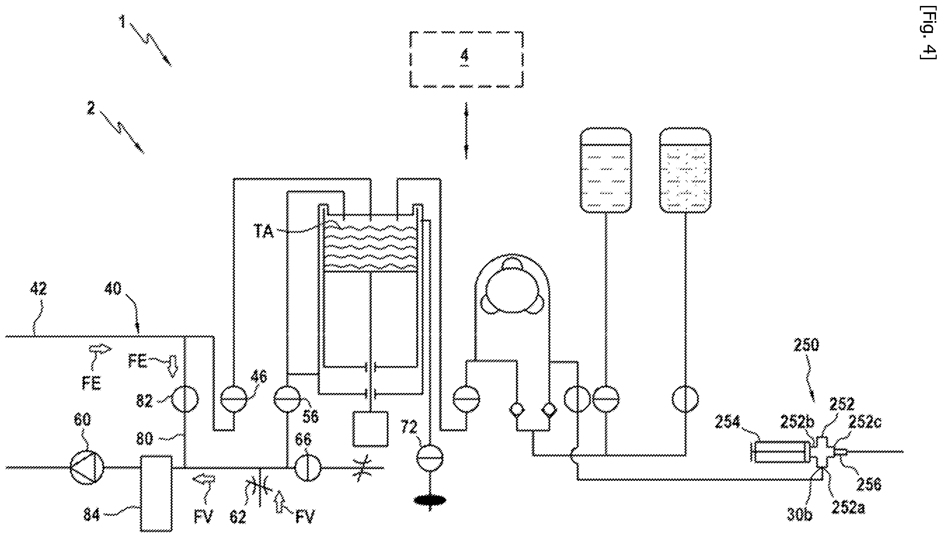

Le lavage du tissu adipeux TA prélevé présent dans le dispositif 100 est ensuite réalisé. On notera toutefois la figure 4 qui illustre une variante où le prélèvement est poursuivi dans le cas où le chirurgien souhaite réaliser une lipoaspiration supplémentaire avec prélèvement d'un excédent de tissu adipeux par rapport à la quantité nécessaire pour réintroduction dans le corps. Dans ce cas, cet excédent n'est pas destiné à passer dans le dispositif 100 mais à être récupéré dans un réservoir 84 de récupération en communication avec la pompe 60. Le réservoir 84 constitue une poubelle et est destiné à récupérer des éléments qui ne sont pas destinés à une introduction dans le patient. Dans le cas illustré à la figure 4, le dispositif de contrôle met les moyens 46 et 56 et l'électrovanne 66 en configuration fermée et ouvre le moyen 82 de contrôle de la circulation de matière lorsque suffisamment de tissu adipeux TA a été prélevé dans le dispositif 100. La pompe 60 est toujours actionnée. Le moyen 82 est présent sur un segment 80 de dérivation qui est placé en dérivation par rapport au dispositif 100 et est apte à être mis en communication avec la pompe 60. Le segment 80 s'étend entre le nœud 45 et le réservoir 84 et l'excédent de tissu adipeux est aspiré par la pompe 60 au travers du conduit 42 et du segment 80 pour être récupéré dans le réservoir 84 sans passer par le dispositif 100 (flèches FE sur la figure 4).

Selon l'exemple illustré, la machine 1 est apte à réaliser le prélèvement du tissu adipeux. Cet aspect est avantageux car il permet que le circuit 2 définisse une boucle de circulation fermée entre le segment 40 et le dispositif 250 avec le corps du patient. Néanmoins selon une variante non préférentielle de l'invention mais couverte par le libellé des revendications, les conduits 42, 44 et 80 peuvent être omis et le remplissage du dispositif 100 par le tissu adipeux prélevé peut être effectué à l'aide d'un dispositif tiers ne faisant pas partie intégrante de la machine en vue de la purification du tissu pour réintroduction dans le corps. La structure d'un exemple de dispositif 100 permettant la purification du tissu adipeux prélevé va maintenant être décrite dans la suite en lien avec les figures 11 à 13. On notera que, dans le cas où un excédent de tissu adipeux est prélevé, une partie au moins de la purification peut être effectuée en même temps que le prélèvement de cet excédent, afin de diminuer le temps de traitement.

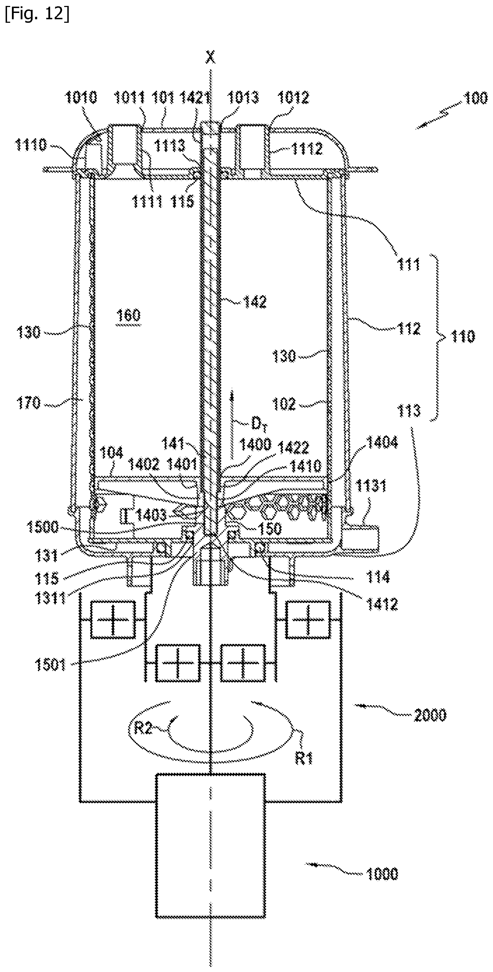

Le dispositif de purification 100 comprend une enceinte étanche 110 formée ici par un capot 111, une paroi latérale 112 et un fond 113. Ces éléments sont fixés ensemble de manière étanche. Les moyens 102 sont présents dans l'enceinte étanche 110. Les moyens 102 délimitent une chambre de centrifugation 160 (visible en figure 12 et correspondant au volume de traitement V décrit précédemment).

Dans l'exemple décrit ici, les moyens 102 présentent une forme cylindrique mais l'homme du métier reconnaîtra que les moyens 102 peuvent présenter d'autres formes adaptées pour la centrifugation. Comme indiqué plus haut, les moyens 102 présentent une taille de pore configurée pour laisser passer un milieu liquide et retenir un tissu adipeux. La taille de pore est choisie en particulier pour permettre le passage de liquides tels que de l'huile, du sang ou une solution physiologique tout en retenant le tissu adipeux. D'une manière générale, la taille des pores des moyens 102 peut être inférieure ou égale à 1,5 mm, par exemple inférieure ou égale à 0,5 mm. Cette taille peut être comprise entre 0,05 mm et 1,5 mm, par exemple entre 0,2 mm et 0,5 mm. Les moyens 102 peuvent être en matériau polymérique, par exemple en polyester ou en polypropylène mais l'homme du métier reconnaîtra que d'autres matériaux peuvent être utilisés.

Dans l'exemple décrit ici, un élément de rigidification 130 est présent entre la paroi 112 de l'enceinte étanche 110 et les moyens 102. Il a notamment pour fonction d'assurer la tenue structurale des moyens 102 pendant la centrifugation. L'élément de rigidification 130 est par exemple réalisé en matériau métallique ou plastique et présente une structure ajourée définissant une pluralité d'ouvertures 1300 afin de permettre l'évacuation du milieu liquide drainé par les moyens 102.

L'élément de rigidification 130 est associé à un plateau rotatif 131. Plus précisément, l'élément de rigidification 130 comporte à son extrémité inférieure des dents 1301 qui coopèrent avec des rainures 1310 présente au voisinage de la périphérie externe du plateau rotatif 131. L'homme du métier reconnaîtra que l'élément 130 peut être relié au plateau 131 de différentes manières. Le plateau rotatif 131 est relié à un moyen d'entrainement en rotation qui peut être manuel ou motorisé. Dans l'exemple décrit ici, le plateau rotatif est relié à un moteur électrique 1000, comme un moteur pas à pas ou un moteur à courant continu sans balais, via un embrayage bidirectionnel 2000 configuré pour assurer l'entrainement en rotation du plateau rotatif 131 suivant un premier sens de rotation R1 (figure 12).

Des joints 114 et 115 sont placés respectivement en-dessous et au-dessus du plateau rotatif 131 afin d'assurer l'étanchéité dans la partie inférieure du dispositif de purification.

La centrifugation est réalisée par mise en rotation des moyens 102. Plus précisément, le moteur électrique 1000 est commandé suivant le premier sens de rotation R1 pour entrainer le plateau rotatif 131 et l'élément de rigidification 130 en prise avec le plateau 131. La mise en rotation du plateau 131 et de l'élément de rigidification 130 entraine la mise en rotation des moyens 102 qui sont solidaires de l'élément de rigidification 130, en étant par exemple collés ou clipsés à ce dernier. La vitesse du moteur électrique 1000 est contrôlée de manière à appliquer une force centrifuge au matériau dans la chambre de centrifugation 160. Ainsi, un tissu adipeux présent dans la chambre de centrifugation 160 sera soumis à une force centrifuge contre la paroi interne des moyens 102, ce qui permet de drainer efficacement le milieu liquide présent dans le tissu adipeux sans abimer celui-ci. Durant la centrifugation, le milieu liquide traversant les moyens 102 et l'élément de rigidification 130 est collecté dans un volume 170 (correspondant au volume d'évacuation VE décrit précédemment) délimité entre l'élément de rigidification 130 et la paroi 112 de l'enceinte 110. Le liquide est ensuite évacué via un port d'évacuation 1131 présent sur le fond 113 de l'enceinte 110, le port 1131 définissant l'orifice 50b décrit plus haut. Dans l'exemple décrit ici, le capot 111 comprend trois ports 1110, 1111 et 1112 destinés à être reliés respectivement à un dispositif d'aspiration de tissu adipeux prélevé depuis le corps d'un patient (conduit 52 décrit plus haut), au conduit 44 pour l'introduction du tissu adipeux prélevé et à un dispositif de délivrance du liquide de lavage (conduit 95 décrit plus bas). Le dispositif de purification comprend un couvercle 101 comportant des ouvertures 1010, 1011 et 1012 qui coopèrent avec les ports 1110, 1111 et 1112 du capot 111.

Le dispositif de purification peut en outre comprendre un fond 104 de la chambre 160 de centrifugation, par exemple sous la forme d'un plateau de collecte, qui est mobile à l'intérieur de la chambre de centrifugation. Comme illustré dans l'exemple des figures 11 et 12, le dispositif de purification 100 comprend en outre un plateau de collecte 104 mobile en translation dans une direction D_{T} suivant l'axe X des moyens 102. Plus précisément, le dispositif de purification 100 comprend une tige filetée 141 qui s'étend verticalement à l'intérieur de la chambre de centrifugation 160 suivant l'axe X des moyens 102. L'extrémité inférieure 1412 de la tige filetée est reliée au moteur électrique 1000 par l'intermédiaire d'un guide 150 et de l'embrayage bidirectionnel 2000. Le guide 150 comprend un logement 1500 dans lequel l'extrémité inférieure 1412 est fixée. Une portion inférieure 1501 du guide 150 est connectée à une partie de l'embrayage bidirectionnel 2000 qui est en prise avec le moteur électrique 1000 que lorsque celui-ci transmet un mouvement de rotation suivant un deuxième sens de rotation R2 opposé au premier sens de rotation R1 utilisé pour la centrifugation. Lorsque le moteur électrique 10 tourne suivant le premier sens de rotation R1, aucun mouvement de rotation n'est transmis par l'embrayage bidirectionnel 2000 au guide 150 auquel est relié la tige filetée 141. Lorsque le moteur 1000 tourne suivant le deuxième sens de rotation R2, aucun mouvement de rotation n'est transmis par l'embrayage bidirectionnel 2000 au plateau rotatif 131 et, par conséquent, aux moyens 102. Le plateau de collecte 104 comporte sur sa face supérieure une ouverture centrale 1400 prolongée par un col 1401 qui s'étend à partir de la face inférieure du plateau 104. Le col 1401 comprend une portion 1402 comportant un taraudage 1403 qui coopère avec un filetage 1411 de la tige filetée 141. Ainsi, lorsque que la tige filetée est entrainée en rotation suivant le deuxième sens de rotation R2, le plateau de collecte 104 s'élève dans la chambre de centrifugation 160 suivant la direction D_{T}.

Le bord périphérique 1404 du plateau de collecte 104 est en vis-à-vis de la paroi interne des moyens 102. Ainsi, lorsque le plateau de collecte 104 est déplacé en translation verticale suivant la direction D_{T}, celui-ci joue un rôle de piston qui permet de racler la paroi interne du filtre de manière à collecter davantage de tissu adipeux et faciliter l'évacuation du tissu adipeux purifié en sommet du dispositif pour introduction dans le corps du patient via le dispositif 250.

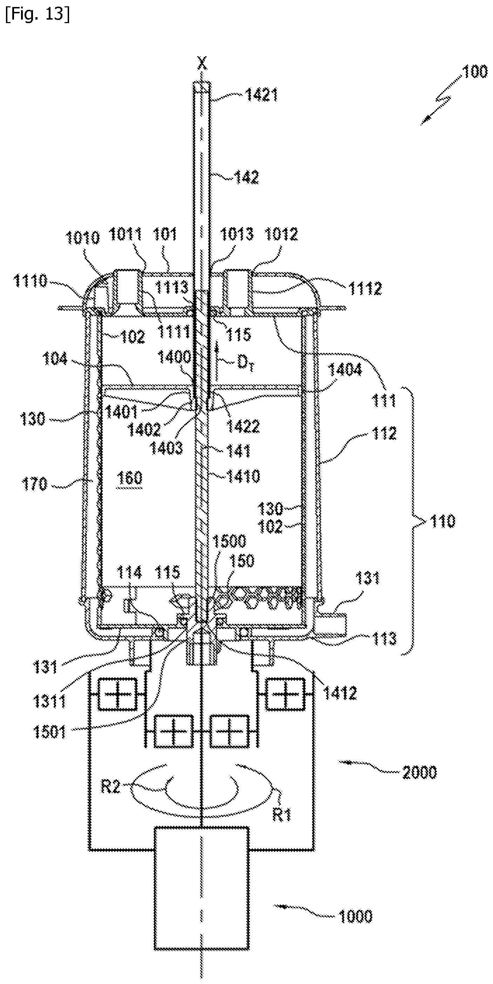

Selon un aspect particulier, la tige filetée 141 peut être logée dans une gaine de protection 142. La gaine de protection 142 qui s'étend entre une extrémité supérieure 1421 et une extrémité inférieure 1422 permet d'éviter à la tige filetée 141 d'entrer en contact avec le tissu adipeux qui en s'accumulant au niveau du taraudage 1403 du plateau de collecte 104 peut bloquer le déplacement de celui-ci. La figure 13 montre le dispositif de purification 100 après actionnement du moteur électrique 1000 dans le deuxième sens de rotation R2 permettant d'entraîner en rotation la tige filetée 141. La rotation de la tige filetée 141 entraîne la translation verticale du plateau de collecte 104 suivant la direction D_{T}.

L'extrémité inférieure 1422 de la gaine de protection est fixée dans l'ouverture 1400 du plateau de collecte 104. Afin de permettre le mouvement de la gaine de protection 142 lors du déplacement du plateau 104, le capot 111 et le couvercle 101 comportent respectivement une ouverture 1113 et une ouverture 1013 au travers desquelles la gaine 142 coulisse. Un joint 115 est présent autour de l'ouverture 1113 afin de préserver l'étanchéité en sommet de la chambre de centrifugation.

Dans un autre exemple de réalisation, les moyens 102 sont en un matériau rigide autoporteur comme par exemple un matériau métallique. Dans ce cas, l'élément de rigidification 130 n'est plus nécessaire et ce sont les moyens 102 autoporteurs qui sont directement en prise avec le plateau rotatif 131.

Selon une variante non illustrée, on notera qu'il est possible d'adjoindre un système de circulation à turbine permettant d'améliorer la circulation des liquides dans le dispositif 100, en particulier du liquide de lavage.

D'une manière générale, le dispositif 100 permet une purification par centrifugation du tissu adipeux TA prélevé. On notera que l'on peut réaliser plusieurs phases de centrifugation à différents moments du procédé et notamment alors que le tissu adipeux TA est prélevé, ou durant l'introduction du liquide de lavage afin de brasser l'ensemble des matériaux présents dans le volume V et éviter tout risque de bouchage des moyens 102 par le tissu adipeux.

D'une manière générale, une phase de centrifugation est effectuée par mise en rotation des moyens 102. Les moyens 102 sont mis en rotation sur eux-mêmes. Les moyens 102 sont mis en rotation autour de l'axe X (voir figure 12). L'axe X peut correspondre à l'axe de la hauteur des moyens 102, et par exemple à un axe de symétrie ou de révolution de ces moyens 102. Du fait de cette rotation, le tissu adipeux et les matières polluantes peuvent subir une accélération supérieure ou égale à 8 G, par exemple supérieure ou égale à 10 G ou supérieure ou égale à 12G. Cette accélération mesurée en G correspond au rapport entre l'accélération subie par le matériau et l'accélération de la pesanteur terrestre, laquelle est d'environ 9,81 m²/s. L'accélération subie par le matériau correspond au rapport de la force centrifuge appliquée et de la masse du matériau considéré. La force centrifuge appliquée est égale à m*ω² * R où m est la masse de l'objet considéré, ω est la vitesse angulaire des moyens 102 exprimée en rad/s et R la distance de l'axe X de rotation au centre de gravité de l'objet considéré. Durant une phase de centrifugation, le tissu adipeux et les matières polluantes peuvent subir une accélération inférieure ou égale à 40 G, par exemple inférieure ou égale à 30 G, voire inférieure ou égale à 25 G ou inférieure ou égale à 20 G. Cette accélération peut être comprise entre 8 G et 40 G ou entre 8 G et 30 G ou entre 8 G et 25 G ou entre 8 G et 20 G. Cette accélération peut être comprise entre 10 G et 40 G ou entre 10 G et 30 G ou entre 10 G et 25 G ou entre 10 G et 20 G. Cette accélération peut être comprise entre 12 G et 40 G ou entre 12 G et 30 G ou entre 12 G et 25 G ou entre 12 G et 20 G. Les valeurs d'accélération décrites plus haut permettent d'optimiser l'élimination des liquides présents dans le tissu adipeux et notamment du liquide interstitiel et améliorer davantage encore la qualité du tissu adipeux purifié. Ce dernier point est plus difficile à réaliser dans le cas d'une filtration par gravité comme décrite dans US 2020/0054824. Par ailleurs, le fait de limiter l'accélération à des valeurs bien inférieures à celles mises en œuvre en centrifugation classique comme décrit ci-dessus, participe également à obtenir un tissu adipeux de qualité optimale et avec une légère quantité de liquide interstitiel résiduel lui permettant d'être facilement injecté dans le corps du patient.

La durée d'un cycle de centrifugation peut typiquement être supérieure ou égale à 10 secondes. Cette durée peut être inférieure ou égale à 60 secondes. Cette durée peut par exemple comprise entre 10 secondes et 60 secondes, par exemple entre 15 secondes et 45 secondes. On notera que l'on peut effectuer plusieurs cycles de centrifugation avec arrêt de la rotation de ces moyens entre ces cycles à différentes étapes du procédé, par exemple une alternance de cycles de centrifugation avec arrêt de la rotation durant le prélèvement du tissu adipeux, ou lors du lavage.

La purification du tissu adipeux prélevé (étape E30) est initiée par le chirurgien comme décrit plus haut et peut donc être initiée pendant le prélèvement du tissu adipeux. La purification comprend notamment le lavage du tissu adipeux qui va à présent être décrit.

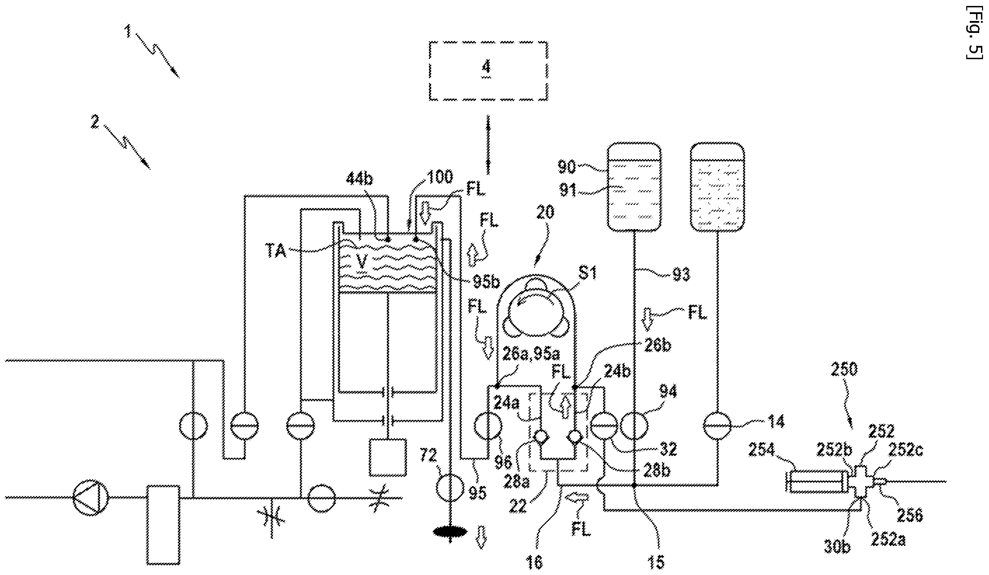

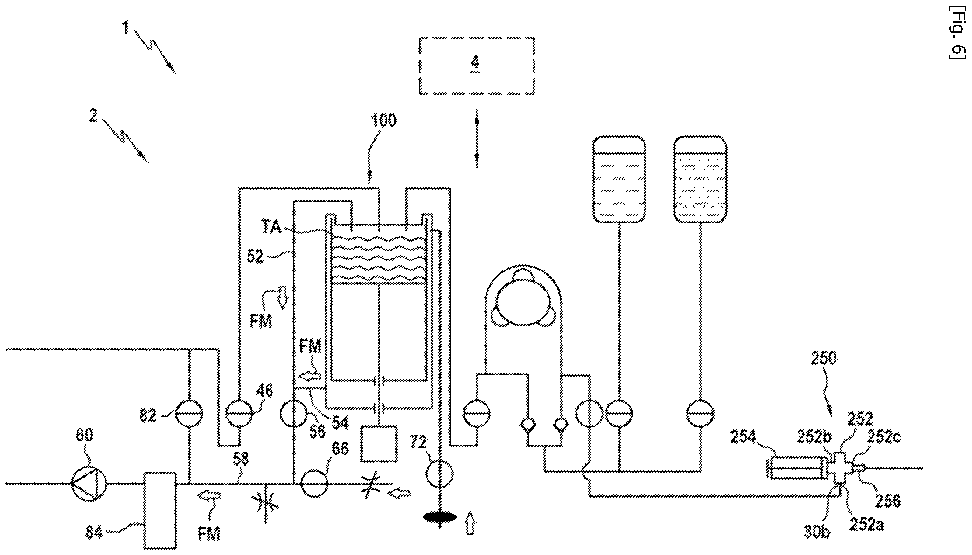

La figure 5 illustre la configuration du circuit 2 permettant le lavage du tissu adipeux. Le circuit 2 comprend un contenant 90 contenant le liquide 91 de lavage. Le liquide 91 peut être une solution physiologique. Le contenant 90 peut être sous la forme d'une poche souple, par exemple en matière plastique. Le contenant 90 est relié à l'entrée 22 de la pompe 20, par l'intermédiaire des conduits 93 et 16 dans l'exemple illustré. Le conduit 93 comprend un moyen de contrôle 94 de la circulation de matière qui est apte à autoriser ou empêcher le transfert de matière depuis le contenant 90 au travers de ce conduit 93. Le circuit 2 comprend également un conduit 95 reliant la pompe 20 au dispositif 100. Le conduit 95 s'étend ici entre une première extrémité 95a au niveau de l'orifice 26a et une deuxième extrémité 95b qui débouche dans le volume V du dispositif 100. Le conduit 95 est distinct du conduit 24a qui s'étend également jusqu'à l'orifice 26a. L'extrémité 95b peut être distincte de l'extrémité 44b décrite précédemment ou, selon une variante non illustrée, les conduits 95 et 44 peuvent être réunis en un même conduit débouchant dans le volume V du dispositif 100. Dans l'exemple illustré, le dispositif 100 est relié à un orifice 26a du corps 21 de pompe 20 qui est distinct de l'orifice 26b auquel est relié le segment 30. Le conduit 95 comprend un moyen de contrôle 96 de la circulation de matière qui est apte à mettre la pompe 20 en communication ou non avec le dispositif 100. Selon la configuration de lavage de la figure 5, les moyens 94, 96 et 72 sont en configuration ouverte et les moyens 14 et 32 en configuration fermée. La pompe 20 est actionnée et provoque un écoulement de matière le long du sens d'écoulement S1, opposé au sens d'écoulement S2 décrit précédemment. Le liquide 91 est aspiré depuis le contenant 90 par la pompe 20 pour être refoulé dans le conduit 95 et être ainsi introduit dans le dispositif 100. Le liquide 91 s'écoule le long du sens d'écoulement S1 dans lequel il traverse les conduits 93 et 16 jusqu'à l'entrée 22 de la pompe 20. Le liquide 91 est aspiré par la pompe 20 au travers du conduit 24b et de l'orifice 26b pour être ensuite refoulé au travers de l'orifice 26a et du conduit 95 vers le dispositif 100. Les flèches FL matérialisent, à la figure 5, l'écoulement du liquide 91 dans le circuit 2 durant le lavage. Durant l'introduction du liquide de lavage dans le volume V, on réalise un ou plusieurs cycles de centrifugation comme décrit plus haut pour séparer les matières polluantes. Une aspiration par la pompe 60 est effectuée ensuite afin d'évacuer le liquide de lavage contenant les matières polluantes (eau, sang, huile etc.) du tissu adipeux et le récupérer dans le réservoir 84. La figure 6 illustre la configuration du circuit 2 permettant cette aspiration. Le moyen 82 est en configuration fermée et les moyens 56 et 72 en configuration ouverte ainsi que l'électrovanne 66. La pompe 60 est actionnée. Dans l'exemple illustré, les matières polluantes (flèches FM) sont aspirées à l'extérieur du dispositif 100 au travers des conduits 52, 54 et 58 pour être récupérées dans le réservoir 84.

Après cette aspiration, on peut réaliser à nouveau, si cela est souhaité, un cycle de centrifugation pour éliminer davantage de matières polluantes. Cette séquence lavage / aspiration / centrifugation peut ensuite être répétée si besoin.

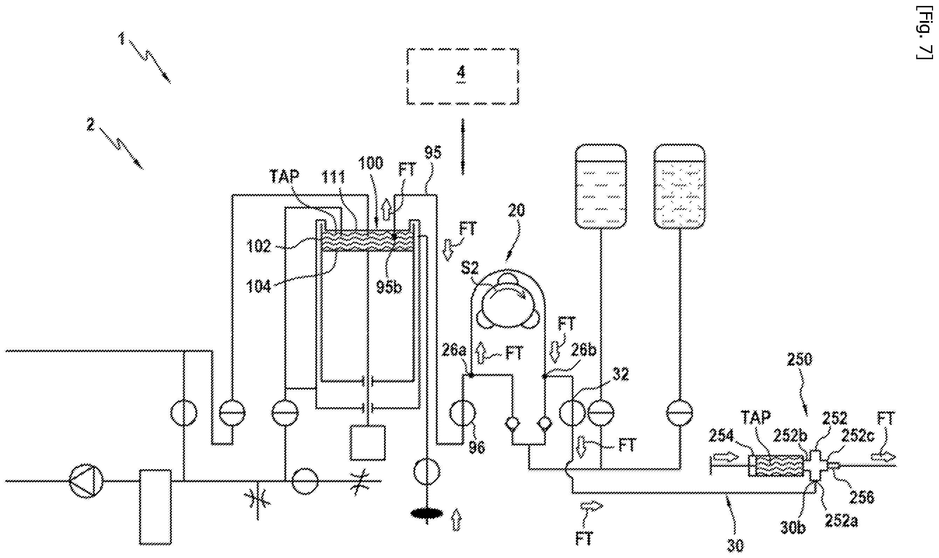

Si cela est souhaité, la purification peut être complétée après les séquences décrites plus haut par un compactage du tissu adipeux dans lequel le fond 104 peut être remonté, tel un piston (voir position du fond 104 en figures 7 et 13), et une aspiration peut être réalisée sur le tissu adipeux compacté, de manière similaire à ce qui vient d'être décrit, pour évacuer l'air et des matières polluantes éventuellement encore présentes.

Une fois la purification terminée, le chirurgien actionne l'étape de réintroduction dans le corps du patient (étape E40 sur la figure 1). Dans l'exemple illustré à la figure 7, le tissu adipeux purifié TAP est introduit dans le segment 30 par la pompe 20 le long du sens d'écoulement S2, opposé à celui utilisé pour l'introduction du liquide 91 dans le dispositif 100. Dans cet exemple, une légère pression est appliquée au tissu adipeux purifié pour le plaquer contre le capot 111 du dispositif 100 du fait de la remontée du fond 104 et procéder à son évacuation vers le segment 30 au travers de l'extrémité 95b. La pompe 20 aspire le tissu adipeux purifié au travers du conduit 95, de l'orifice 26a et le tissu adipeux purifié TAP est ensuite refoulé au travers de l'orifice 26b pour être introduit dans le segment 30 pour introduction dans le dispositif 250 qui va permettre la réintroduction du tissu purifié TAP dans le patient. Les flèches FT sur la figure 7 matérialisent le transfert du tissu adipeux purifié et sa réintroduction dans le patient par le dispositif 250.

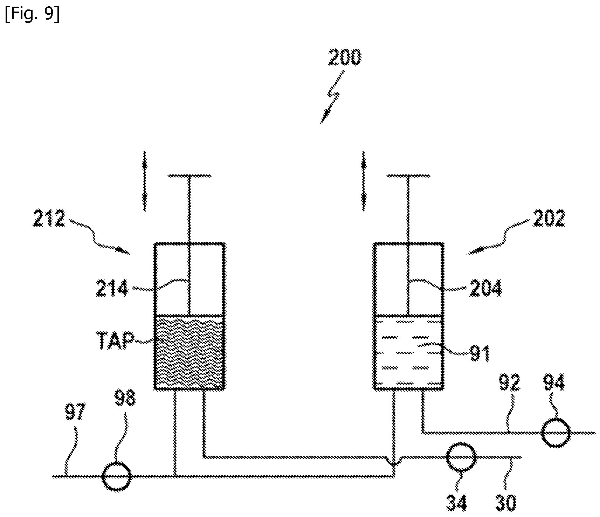

La description qui vient d'être faite, en lien avec les figures 2 à 8, concerne un exemple où la pompe de circulation est une pompe péristaltique. L'invention n'est toutefois pas limitée à une telle solution. Une variante est illustrée, à la figure 9, où la pompe 200 de circulation est formée par un système multi-seringues. Selon cette variante, la pompe 200 comprend au moins une première seringue 202 à piston 204 qui est configurée pour aspirer le liquide de lavage 91 notamment au travers du conduit 92 pour l'injecter ensuite dans le dispositif 100 par transfert dans le conduit 97 pour réaliser le lavage du tissu adipeux décrit plus haut. La pompe 200 comprend une deuxième seringue 212 à piston 214 qui est configurée pour aspirer le tissu adipeux purifié TAP depuis le dispositif 100 au travers du conduit 97 pour l'injecter ensuite dans le conduit de sortie 30 puis dans le dispositif 250 pour réintroduction dans le corps du patient. On a représenté un cas où les conduits 92 et 97 ont des points d'entrée distincts dans la seringue 202 (de même pour les conduits 97 et 30 dans la seringue 212) mais on ne sort pas du cadre de l'invention lorsque ces conduits débouchent dans la seringue au travers d'un orifice commun. On a représenté à la figure 9 le cas d'un système multi-seringues mais on pourrait également utiliser une pompe de circulation formée d'une seringue unique.

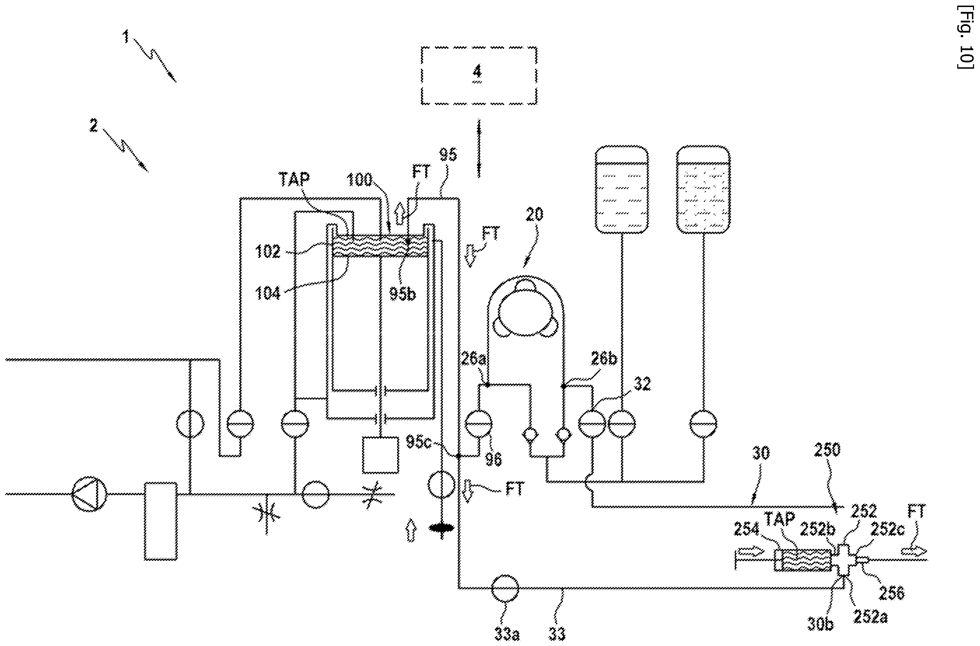

Selon une autre variante décrite à la figure 10, l'introduction du tissu adipeux purifié TAP dans le dispositif 250 n'est pas réalisée en utilisant la pompe 20. En effet, le conduit 95 présente un nœud 95c à partir duquel s'étend un conduit 33 distinct du conduit 30 et muni d'un moyen de contrôle de l'écoulement 33a, ici en position ouverte. Le conduit 33 relie le conduit 95 au dispositif 250 pour la réintroduction du tissu purifié TAP dans le corps du patient. Il n'est donc pas nécessaire d'utiliser la pompe 20 pour la réintroduction du tissu adipeux purifié TAP dans le corps du patient.

L'expression « compris(e) entre ... et ... » doit se comprendre comme incluant les bornes.

## Revendications

1. Machine (1) pour la purification d'un tissu adipeux (TA) et l'introduction du tissu adipeux purifié (TAP) dans le corps d'un patient, la machine comprenant au moins :
- un circuit (2) de circulation de matière comprenant au moins (i) un dispositif (100) de purification apte à purifier un tissu adipeux prélevé du corps d'un patient, le dispositif de purification comprenant des moyens (102) de rétention du tissu adipeux (TA) et de filtration des matières polluantes délimitant un volume de traitement (V) destiné à accueillir le tissu adipeux, et un moyen d'entrainement en rotation apte à mettre les moyens de rétention et de filtration en rotation, et (ii) un dispositif d'introduction (250) apte à être mis en communication avec le dispositif de purification et destiné à l'introduction du tissu adipeux purifié dans le corps du patient, le circuit étant en outre muni de moyens (32 ; 94 ; 96) de contrôle de la circulation de matière aptes à autoriser ou empêcher la communication entre des éléments du circuit, et
- une unité de commande (4) configurée pour actionner le dispositif de purification et les moyens de contrôle,
l'unité de commande étant configurée pour mettre le circuit dans une configuration de purification du tissu adipeux dans laquelle les moyens de rétention et de filtration sont mis en rotation pour purifier le tissu adipeux par centrifugation et filtration des matières polluantes, et l'unité de commande étant configurée pour mettre le circuit dans une configuration d'introduction du tissu adipeux purifié dans le corps du patient dans laquelle le tissu adipeux purifié est introduit depuis le dispositif de purification dans le dispositif d'introduction,
dans laquelle le circuit comprend, en outre, un segment d'entrée (40) destiné au prélèvement du tissu adipeux du corps du patient qui est relié au dispositif de purification et une pompe (60) de prélèvement configurée pour introduire le tissu adipeux prélevé depuis le segment d'entrée dans le dispositif de purification, le circuit étant apte à définir une boucle de circulation fermée entre le segment d'entrée et le dispositif d'introduction avec le corps du patient,
l'unité de commande étant configurée pour actionner la pompe de prélèvement et mettre le circuit dans une configuration de prélèvement du tissu adipeux du corps du patient dans laquelle le tissu adipeux prélevé est introduit dans le dispositif de purification par la pompe de prélèvement, et retenu par les moyens de rétention et de filtration dans le dispositif de purification,
dans laquelle le segment d'entrée est apte à communiquer avec une entrée d'aspiration (44b) du dispositif de purification et le circuit comprend, en outre, un réservoir (84) de récupération relié à au moins une sortie d'aspiration (50a ; 50b) du dispositif de purification et apte à être mis en communication avec la pompe de prélèvement,
l'unité de commande étant configurée pour mettre le circuit dans une configuration de purification dans laquelle des matières polluantes du tissu adipeux sont aspirées par la pompe de prélèvement au travers de ladite au moins une sortie d'aspiration du dispositif de purification et récupérées dans le réservoir de récupération,
dans laquelle le réservoir de récupération est relié au segment d'entrée par un segment (80) de dérivation, placé en dérivation par rapport au dispositif de purification, et apte à être mis en communication avec la pompe de prélèvement,
l'unité de commande étant configurée pour mettre le circuit dans une configuration de prélèvement d'un excédent de tissu adipeux du corps du patient dans laquelle la pompe de prélèvement aspire l'excédent de tissu adipeux au travers du segment de dérivation pour le récupérer dans le réservoir de récupération.

2. Machine (1) selon la revendication 1, dans laquelle les moyens (102) de rétention et de filtration sont présents entre le volume de traitement et un volume (VE) d'évacuation distinct du volume de traitement, et dans laquelle le réservoir (84) de récupération est relié à une première (50a) et une deuxième (50b) sorties d'aspiration du dispositif (100) de purification, la première sortie d'aspiration débouchant dans le volume de traitement et la deuxième sortie d'aspiration débouchant dans le volume d'évacuation.

3. Machine (1) selon l'une quelconque des revendications 1 ou 2,
dans laquelle le circuit comprend en outre un contenant (90) de stockage destiné à contenir un liquide (91) de lavage du tissu adipeux, et une pompe (20) de circulation apte à mettre en communication le contenant de stockage avec le dispositif de purification,
l'unité de commande étant configurée pour mettre le circuit dans une configuration de lavage du tissu adipeux dans laquelle le liquide de lavage est introduit depuis le contenant de stockage dans le dispositif de purification par la pompe de circulation.

4. Machine (1) selon la revendication 3, dans laquelle le circuit (2) comprend en outre un contenant (10) de stockage additionnel destiné à contenir un anesthésiant (11), la pompe de circulation étant apte à mettre en communication le contenant de stockage additionnel avec un segment (30) de sortie destiné à l'introduction de matière dans le corps du patient,
l'unité de commande (4) étant configurée pour mettre le circuit dans une configuration d'anesthésie dans laquelle l'anesthésiant est introduit depuis le contenant de stockage additionnel dans le segment de sortie par la pompe de circulation.

5. Machine (1) selon la revendication 4, dans laquelle la pompe (20) de circulation est une pompe à écoulement réversible apte à mettre en communication le contenant (90) de stockage avec le dispositif (100) de purification selon un premier sens (S1) d'écoulement, et le contenant (10) de stockage additionnel avec le segment (30) de sortie selon un deuxième sens (S2) d'écoulement différent du premier sens d'écoulement.

6. Machine (1) selon l'une quelconque des revendications 3 à 5, dans laquelle la pompe (20) de circulation est une pompe péristaltique.

7. Ensemble pour la purification d'un tissu adipeux (TA) et l'introduction du tissu adipeux purifié (TAP) dans le corps d'un patient, comprenant au moins :
- une machine (1) selon l'une quelconque des revendications 1 à 6, et
- un dispositif chirurgical (256) apte à introduire le tissu adipeux purifié dans le corps du patient relié au dispositif d'introduction (250).

8. Ensemble selon la revendication 7, dans lequel l'ensemble comprend en outre un dispositif chirurgical apte à prélever le tissu adipeux (TA) relié au segment (40) d'entrée.

## Patentansprüche

1. Maschine (1) zur Reinigung eines Fettgewebes (TA) und zur Einbringung des gereinigten Fettgewebes (TAP) in den Körper eines Patienten, wobei die Maschine zumindest umfasst:
- einen Kreis (2) zur Zirkulation von Material, umfassend zumindest (i) eine Vorrichtung (100) zur Reinigung, die dazu geeignet ist, ein aus dem Körper eines Patienten entnommenes Fettgewebe zu reinigen, wobei die Vorrichtung zur Reinigung Einrichtungen (102) zum Zurückhalten des Fettgewebes (TA) und zum Filtern der verunreinigenden Stoffe, die ein Behandlungsvolumen (V) begrenzen, das dazu bestimmt ist, das Fettgewebe aufzunehmen, und eine Einrichtung zum Drehantrieb umfasst, die dazu geeignet ist, die Rückhalte- und Filtrationseinrichtungen in Drehung zu versetzen, und (ii) eine Vorrichtung zur Einbringung (250), die dazu geeignet ist, mit der Vorrichtung zur Reinigung in Kommunikation versetzt zu werden und zur Einbringung des gereinigten Fettgewebes in den Körper des Patienten bestimmt ist, wobei der Kreis ferner mit Einrichtungen (32; 94; 96) zur Steuerung der Zirkulation von Material ausgestattet ist, die dazu geeignet sind, die Kommunikation zwischen den Elementen des Kreises zuzulassen oder zu verhindern, und
- eine Steuereinheit (4), die dazu konfiguriert ist, die Vorrichtung zur Reinigung und die Einrichtungen zur Steuerung zu betätigen,
wobei die Steuereinheit dazu konfiguriert ist, den Kreis in eine Konfiguration zur Reinigung des Fettgewebes zu versetzen, in welcher die Rückhalte- und Filtrationseinrichtungen in Drehung versetzt werden, um das Fettgewebe durch Zentrifugation und Filtration von verunreinigenden Stoffen zu reinigen, und wobei die Steuereinheit dazu konfiguriert ist, den Kreis in eine Konfiguration zur Einbringung des gereinigten Fettgewebes in den Körper des Patienten zu versetzen, in welcher das gereinigte Fettgewebe aus der Vorrichtung zur Reinigung in die Vorrichtung zur Einbringung eingebracht wird,
wobei der Kreis ferner ein Eingabesegment (40), das zur Entnahme des Fettgewebes aus dem Körpers des Patienten bestimmt ist und mit der Vorrichtung zur Reinigung verbunden ist, und eine Pumpe (60) zur Entnahme umfasst, die dazu konfiguriert ist, das entnommene Fettgewebe aus dem Eingabesegment in die Vorrichtung zur Reinigung einzubringen, wobei der Kreis dazu geeignet ist, zwischen dem Eingabesegment und der Vorrichtung zur Einbringung mit dem Körper des Patienten eine geschlossene Zirkulationsschleife zu bilden,
wobei die Steuereinheit dazu konfiguriert ist, die Entnahmepumpe zu betätigen und den Kreis in eine Konfiguration zur Entnahme des Fettgewebes aus dem Körper des Patienten zu versetzen, in welcher das entnommene Fettgewebe durch die Entnahmepumpe in die Vorrichtung zur Reinigung eingebracht und durch die Rückhalte- und Filtrationseinrichtungen in der Vorrichtung zur Reinigung zurückgehalten wird,
wobei das Eingabesegment dazu geeignet ist, mit einem Absaugeingang (44b) der Vorrichtung zur Reinigung zu kommunizieren, und der Kreis ferner einen Sammelbehälter (84) umfasst, der mit zumindest einem Absaugausgang (50a; 50b) der Vorrichtung zur Reinigung verbunden und dazu geeignet ist, mit der Entnahmepumpe in Kommunikation versetzt zu werden,
wobei die Steuereinheit dazu konfiguriert ist, den Kreis in eine Konfiguration zur Reinigung zu versetzen, in welcher verunreinigende Stoffe des Fettgewebes durch die Entnahmepumpe über den zumindest einen Absaugausgang der Vorrichtung zur Reinigung abgesaugt und in dem Sammelbehälter gesammelt werden,
wobei der Sammelbehälter mit dem Eingabesegment durch ein Umleitungssegment (80) verbunden ist, das als Umleitung in Bezug auf die Vorrichtung zur Reinigung platziert und dazu geeignet ist, mit der Entnahmepumpe in Kommunikation versetzt zu werden,
wobei die Steuereinheit dazu konfiguriert ist, den Kreis in eine Konfiguration zur Entnahme eines Überschusses an Fettgewebe aus dem Körper des Patienten zu versetzen, in welcher die Entnahmepumpe den Überschuss an Fettgewebe über das Umleitungssegment absaugt und in dem Sammelbehälter sammelt.

2. Maschine (1) nach Anspruch 1, wobei die Rückhalte- und Filtrationseinrichtungen (102) zwischen dem Behandlungsvolumen und einem Ableitungsvolumen (VE) vorhanden sind, das sich von dem Behandlungsvolumen unterscheidet, und wobei der Sammelbehälter (84) mit einem ersten (50a) und einem zweiten (50b) Absaugausgang der Vorrichtung (100) zur Reinigung verbunden ist, wobei der erste Absaugausgang in das Behandlungsvolumen mündet und der zweite Absaugausgang in das Ableitungsvolumen mündet.

3. Maschine (1) nach einem der Ansprüche 1 oder 2, wobei der Kreis ferner einen Speicherbehälter (90), der dazu bestimmt ist, eine Flüssigkeit (91) zum Waschen des Fettgewebes zu enthalten, und eine Zirkulationspumpe (20) umfasst, die dazu geeignet ist, den Speicherbehälter mit der Vorrichtung zur Reinigung in Kommunikation zu versetzen,
wobei die Steuereinheit dazu konfiguriert ist, den Kreis in eine Konfiguration zum Waschen des Fettgewebes zu versetzen, in welcher die Waschflüssigkeit durch die Zirkulationspumpe aus dem Speicherbehälter in die Vorrichtung zur Reinigung eingebracht wird.

4. Maschine (1) nach Anspruch 3, wobei der Kreis (2) ferner einen zusätzlichen Speicherbehälter (10) umfasst, der dazu bestimmt ist, ein Anästhetikum (11) zu enthalten, wobei die Zirkulationspumpe dazu geeignet ist, den zusätzlichen Speicherbehälter mit einem Ausgabesegment (30) in Kommunikation zu versetzen, das zur Einbringung von Material in den Körper des Patienten bestimmt ist,
wobei die Steuereinheit (4) dazu konfiguriert ist, den Kreis in eine Konfiguration zur Anästhesierung zu versetzen, in welcher das Anästhetikum durch die Zirkulationspumpe aus dem zusätzlichen Speicherbehälter in das Ausgabesegment eingebracht wird.

5. Maschine (1) nach Anspruch 4, wobei die Zirkulationspumpe (20) eine Pumpe mit umkehrbarer Strömung ist, die dazu geeignet ist, den Speicherbehälter (90) mit der Vorrichtung (100) zur Reinigung in einer ersten Strömungsrichtung (S1) in Kommunikation zu versetzen, und den zusätzlichen Speicherbehälter (10) mit dem Ausgabesegment (30) in einer zweiten Strömungsrichtung (S2), die sich von der ersten Strömungsrichtung unterscheidet, in Kommunikation zu versetzen.

6. Maschine (1) nach einem der Ansprüche 3 bis 5, wobei die Zirkulationspumpe (20) eine Peristaltikpumpe ist.

7. Anordnung zur Reinigung eines Fettgewebes (TA) und zur Einbringung des gereinigten Fettgewebes (TAP) in den Körper eines Patienten, umfassend zumindest:
- eine Maschine (1) nach einem der Ansprüche 1 bis 6, und
- eine chirurgische Vorrichtung (256), die dazu geeignet ist, das gereinigte Fettgewebe in den Körper des Patienten einzubringen, und mit der Vorrichtung zur Einbringung (250) verbunden ist.

8. Anordnung nach Anspruch 7, wobei die Anordnung ferner eine chirurgische Vorrichtung umfasst, die dazu geeignet ist, das Fettgewebe (TA) zu entnehmen, und mit dem Eingabesegment (40) verbunden ist.

## Claims

1. A machine (1) for the purification of adipose tissue (TA) and introduction of the purified tissue (TAP) into a patient's body, the machine comprising:
- a material circulation circuit (2) comprising at least (i) a purification device (100) able to purify adipose tissue removed from the patient's body, the purification device comprising means (102) for retaining adipose tissue (TA) and filtering contaminants delimiting a treatment volume (V) intended to receive the adipose tissue, and a rotational drive means able to rotate the retaining and filtering means, and (ii) an introduction device (250) able to communicate with the purification device for introducing purified adipose tissue into the patient's body, the circuit being further provided with means (32 ; 94 ; 96) for controlling the circulation of material able to allow or prevent communication between elements of the circuit, and
- a control unit (4) configured to actuate the purification device and the control means,
the control unit being configured to place the circuit in an adipose tissue purification configuration in which the retaining and filtering means are rotated to purify adipose tissue by centrifugation and filtration of contaminants, and the control unit being configured to place the circuit in a configuration for introducing purified adipose tissue into the patient's body in which purified adipose tissue is introduced from the purification device into the introduction device,
wherein the circuit further comprises an inlet segment (40) intended to collect adipose tissue from the patient's body that is connected to the purification device and a removal pump (60) configured to introduce adipose tissue removed from the inlet segment into the purification device, the circuit being able to define a closed circulation loop between the inlet segment and the introduction device with the patient's body,
the control unit being configured to actuate the removal pump and place the circuit in a configuration for removing adipose tissue from the patient's body in which the removed adipose tissue is introduced into the purification device by the removal pump, and retained by the retaining and filtering means in the purification device, wherein the inlet segment is able to communicate with a suction inlet (44b) of the purification device and the circuit further comprises a recovery tank (84) connected to at least one suction outlet (50a; 50b) of the purification device and able to be placed into communication with the removal pump,
the control unit being configured to place the circuit in a purification configuration in which adipose tissue contaminants are suctioned by the removal pump through the at least one suction outlet of the purification device and collected in the recovery tank,
wherein the recovery tank is connected to the inlet segment by a bypass segment (80), placed in bypass with respect to the purification device, and able to be placed into communication with the removal pump,
the control unit is configured to place the circuit in a configuration for collecting excess adipose tissue from the patient's body, wherein the removal pump suctions excess adipose tissue through the bypass segment to recover it into the recovery tank.

2. The machine (1) according to claim 1, wherein the retaining and filtering means (102) are present between the treatment volume and a discharge volume (VE) distinct from the treatment volume, and wherein the recovery tank (84) is connected to a first (50a) and a second (50b) suction outlet of the purification device (100), the first suction outlet opening into the treatment volume and the second suction outlet opening into the discharge volume.

3. The machine according to claim 1 or 2, wherein the circuit further comprises a storage container (90) for containing a liquid (91) for washing the adipose tissue, and a circulation pump (20) able to place the storage container in communication with the purification device,
the control unit being configured to place the circuit in an adipose tissue wash configuration in which wash liquid is introduced from the storage container into the purification device by the circulation pump.

4. The machine (1) according to claim 3, wherein the circuit (2) further comprises an additional storage container (10) for containing an anaesthetic (11), the circulation pump being able to place the additional storage container in communication with an outlet segment (30) for introducing material into the patient's body,
the control unit (4) being configured to place the circuit in an anaesthetic configuration in which anaesthetic is introduced from the additional storage container into the outlet segment by the circulation pump.

5. The machine (1) according to claim 4, wherein the circulation pump (20) is a reversible flow pump able to place the storage container (90) in communication with the purification device (100) along a first flow direction (S1), and the additional storage container (10) with the outlet segment (30) along a second flow direction (S2) different from the first flow direction.

6. The machine (1) according to any one of claims 3 to 5, wherein the circulation pump (20) is a peristaltic pump.

7. An assembly for the purification of adipose tissue (TA) and introduction of the purified tissue (TAP) into a patient's body, comprising:
- a machine (1) according to any one of claims 1 to 6, and
- a surgical device (256) able to introduce purified adipose tissue into the patient's body connected to the introduction device (250).

8. The assembly according to claim 7, wherein the assembly further comprises a surgical device able to remove adipose tissue (TA) connected to the inlet segment (40).
